# FASCICULE DE BREVET EUROPEEN

(11) **EP 2 166 004 B1**
(45) Date de publication et mention de la délivrance du brevet: **06.10.2010**
(21) Numéro de dépôt: 09290646.0
(22) Date de dépôt: 27.08.2009
(51) Int. Cl.: C07D 223/16

(54) **Nouveau procédé de résolution des énantiomères du (3,4-diméthoxy-bicyclo[4.2.0]octa-1,3,5-trién-7-yl)nitrile et application à la synthèse de l'ivabradine**
Neues Auflösungsverfahren von Enantiomeren von (3,4-Dimethoxy-bicyclo[4.2.0]octa-1,3,5-trien-7-yl)Nitril und Anwendung für die Synthese von Ivabradin
New method for resolution of enantiomers of (3,4-dimethoxy-bicyclo[4.2.0]octa-1,3,5-trien-7-yl)nitrile and their application to the synthesis of ivabradine

(30) Priorité: 29.08.2008 FR 0804755
(43) Date de publication de la demande: 24.03.2010
(73) Titulaire: Les Laboratoires Servier, 92284 Suresnes Cedex (FR)
(72) Inventeur: Lerestif, Jean-Michel, 76190 Yvetot (FR); Lecouve, Jean-Pierre, 76600 Le Havre (FR); Dron, Daniel, 76119 sainte Marguerite sur Mer (FR); Gojon, Eric, 76110 Gonfreville-Caillot (FR); Phan, Maryse, 76600 Le Havre (FR)

(56) Documents cités:
- EP-A- 0 534 859
- WO-A-2005/110993
- US-A1- 2005 261 376
- PERRIN C ET AL: "Screening approach for chiral separation of pharmaceuticals - Part I. Normal-phase liquid chromatography" JOURNAL OF CHROMATOGRAPHY, ELSEVIER SCIENCE PUBLISHERS B.V. AMSTERDAM, NL, vol. 947, no. 1, 15 février 2002 (2002-02-15), pages 69-83, XP004334296 ISSN: 0021-9673

## Description

La présente invention concerne un procédé de résolution optique du (3,4-diméthoxybicyclo[4.2.0]octa-1,3,5-trién-7-yl)nitrile de formule (I), et son application à la synthèse de l'ivabradine, de ses sels d'addition à un acide pharmaceutiquement acceptable et de leurs hydrates.

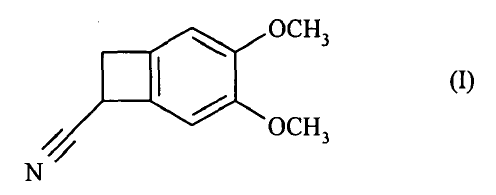

L'ivabradine de formule (II) : 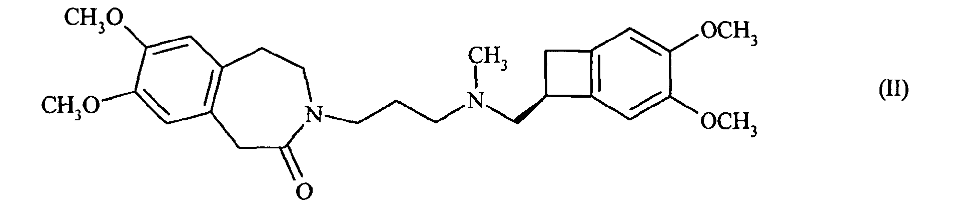 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse de l'ivabradine à partir du composé de formule (III) :

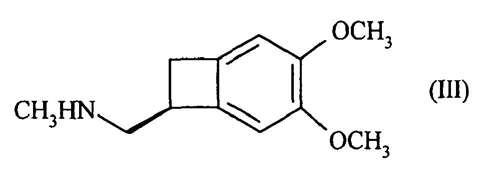

Le composé de formule (III) est préparé à partir du composé de formule (IV) : 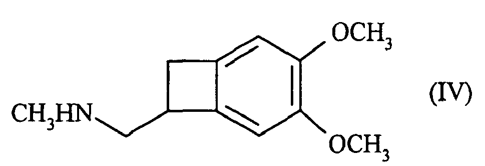 par dédoublement à l'aide de l'acide camphosulfonique.

Le composé de formule (III) est un intermédiaire important dans la synthèse de l'ivabradine.

Le dédoublement de l'amine secondaire de formule (IV) ne conduit au composé de formule (III) qu'avec un faible rendement (4 à 5%).

Or, compte-tenu de l'intérêt pharmaceutique de l'ivabradine et de ses sels, il était impératif de pouvoir accéder au composé de formule (III) avec un procédé industriel performant, et notamment avec un bon rendement et une excellente pureté chimique et énantiomérique.

La Demanderesse a mis au point un procédé de résolution optique du composé de formule (I) permettant d'accéder au composé de formule (III) avec de bons critères de rendement et de pureté chimique et énantiomérique. Le procédé de l'invention permet d'obtenir l'énantiomère cible du composé de formule (I) avec un excellent excès énantiomérique, une forte productivité et un excellent rendement en économisant les solvants utilisés.

Plus spécifiquement, la présente invention concerne un procédé de résolution optique du (3,4-diméthoxy-bicyclo[4.2.0]octa-1,3,5-trién-7-yl)nitrile de formule (I) : 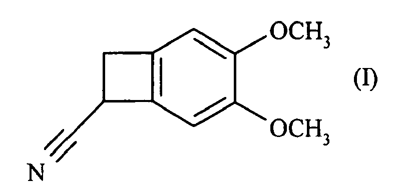 pour conduire à ses énantiomères, respectivement de configuration absolue (*S*) et (*R*), de formule (Ia) et (Ib) : 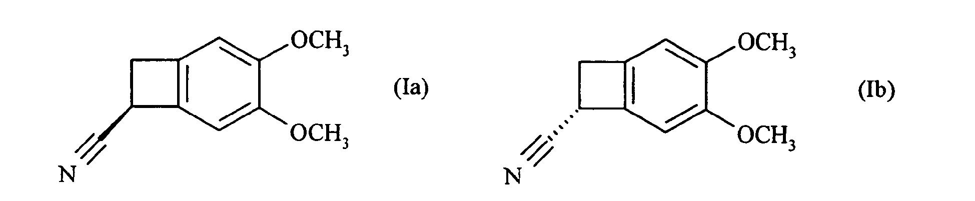 dans lequel le mélange racémique ou énantiomériquement enrichi du (3,4-diméthoxy-bicyclo[4.2.0]octa-1,3,5-trién-7-yl)nitrile est séparé en ses deux énantiomères (*S*)-(3,4-diméthoxy-bicyclo[4.2.0]octa-1,3,5-trién-7-yl)nitrile de formule (Ia) et (*R*)-(3,4-diméthoxy-bicyclo[4.2.0]octa-1,3,5-trién-7-yl)nitrile de formule (Ib) par chromatographie chirale.

Par résolution optique, on entend la séparation des deux énantiomères d'un mélange racémique ou d'un quelconque mélange de ces deux énantiomères.

Par mélange racémique, on entend un mélange de deux énantiomères dans un rapport 55:45 à 45:55, préférentiellement dans un rapport 50:50.

Par mélange énantiomériquement enrichi, on entend un mélange de deux énantiomères contenant significativement plus de l'un des énantiomères dans un rapport variant entre 55:45 et 90:10.

Par chromatographie chirale, on entend le dispositif permettant la séparation des énantiomères d'un mélange au moyen d'une phase stationnaire chirale et d'une phase mobile composée d'un solvant ou d'un mélange de solvants.

Selon un mode de réalisation préféré de l'invention, on utilise un procédé de séparation multi-colonnes en continu.

Selon un mode de réalisation encore plus préféré de l'invention, on utilise un procédé de chromatographie en lit mobile simulé.

Par chromatographie en lit mobile simulé, on entend un procédé de chromatographie en continu permettant de simuler le mouvement de la phase stationnaire dans la direction opposée au mouvement de la phase mobile. Un tel procédé permet de séparer des composés difficiles ou impossibles à séparer par les techniques de chromatographie classiques. Lorsqu'il utilise une phase stationnaire chirale, un tel procédé est particulièrement utile pour la séparation d'énantiomères. L'utilisation de la chromatographie en lit mobile simulé permet d'effectuer la résolution d'un mélange d'énantiomères en continu avec une forte productivité, tout en réduisant les quantités de phases stationnaire et mobile utilisées par rapport à des processus de chromatographie discontinus.

Selon l'un des modes de réalisation préféré de l'invention, la phase stationnaire utilisée pour la chromatographie chirale comprend un gel de silice imprégné par un polysaccharide fonctionnalisé.

Selon un mode de réalisation préféré de l'invention, la phase stationnaire utilisée pour la chromatographie chirale comprend un dérivé cellulosique ou amylosique de tris (4-méthylbenzoate) ou de tris (3,5-diméthylphénylcarbamate).

La phase mobile préférentiellement utilisée pour la chromatographie chirale comprend un alcool, un autre solvant organique ou un mélange d'un alcool et d'un autre solvant organique.

Parmi les alcools pouvant être utilisés pour la chromatographie chirale, on peut citer à titre non limitatif l'isopropanol, l'éthanol ou le méthanol.

L'alcool préférentiellement utilisé pour la chromatographie chirale est l'isopropanol.

Parmi les solvants organiques pouvant être utilisés pour la chromatographie chirale, on peut citer à titre non limitatif l'heptane, l'hexane, le cyclohexane, l'acétonitrile ou l'éther de *tert*-butyle et de méthyle.

Le solvant organique préférentiellement utilisé est l'heptane ou l'hexane.

La phase mobile utilisée pour la chromatographie chirale comprend préférentiellement un mélange d'un alcool et d'un autre solvant organique.

La phase mobile encore plus préférentiellement utilisée pour la chromatographie chirale comprend un mélange d'isopropanol et d'heptane ou un mélange d'isopropanol et d'hexane.

Dans un mode de réalisation préféré de l'invention, la phase mobile utilisée pour la chromatographie chirale comprend un mélange d'isopropanol et d'heptane ou un mélange d'isopropanol et d'hexane dans un rapport variant entre 50:50 et 2:98.

Selon un mode de réalisation préféré de l'invention, la phase mobile utilisée pour la chromatographie chirale est recyclée.

La chromatographie chirale est préférentiellement réalisée à une température comprise entre 15°C et 40°C

Selon un mode de réalisation préféré de l'invention, la résolution optique s'effectue sur le mélange racémique du (3,4-diméthoxy-bicyclo[4.2.0]octa-1,3,5-trién-7-yl)nitrile de formule (I).

Selon l'un des modes de réalisation préféré de l'invention, l'énantiomère du (3,4-diméthoxy-bicyclo[4.2.0]octa-1,3,5-trién-7-yl)nitrile ciblé est le (*S*)-(3,4-diméthoxy-bicyclo[4.2.0]octa-1,3,5-trién-7-yl)nitrile de formule (Ia).

Selon l'un des modes de réalisation préféré de l'invention, l'énantiomère (*R*) du (3,4-diméthoxy-bicyclo[4.2.0]octa-1,3,5-trién-7-yl)nitrile de formule (Ib) est racémisé et utilisé comme matière première dans le procédé de résolution optique.

Le composé de formule (Ia) peut conduire au composé de formule (V): 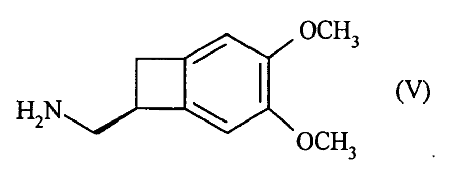 par une réaction de réduction.

La réduction du composé de formule (Ia) est préférentiellement effectuée en présence de palladium sur charbon et d'acide chlorhydrique sous atmosphère d'hydrogène ou en présence de tétraborohydrure de sodium et d'acide trifluoroacétique.

Le composé de formule (V) obtenu par réduction du composé de formule (Ia) est utile dans la synthèse de l'ivabradine de formule (II).

A titre d'exemple, le composé de formule (V) est transformé en carbamate de formule (VI) : 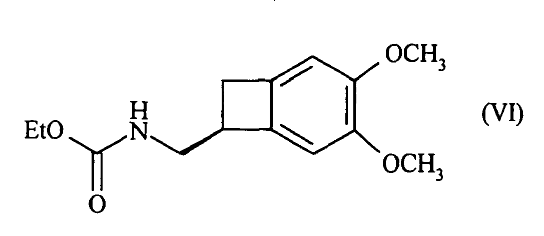 qui est réduit en composé de formule (III) : 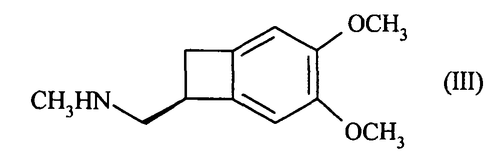 qui est transformé en ivabradine de formule (II) 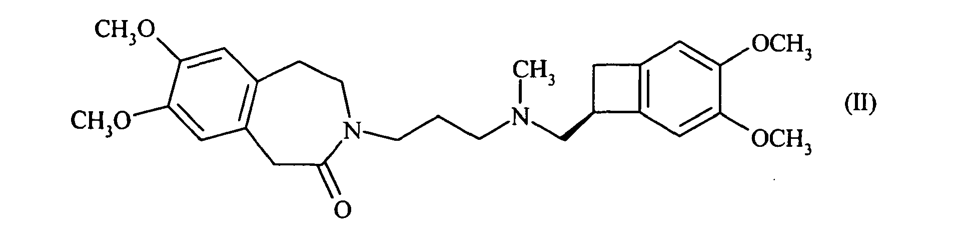 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Parmi les méthodes connues pour effectuer la transformation du composé de formule (III) en ivabradine, on peut citer celles décrites dans les brevets européens EP 0 534 859 et EP 1 589 005.

Les composés de formule (Ia) et (Ib) sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

### Liste des abréviations utilisées :

DBU : 1,8-diazabicyclo[5.4.0]undec-7-ène
éq. : équivalent
TFA : acide trifluoroacétique
THF : tétrahydrofurane

Les exemples ci-dessous illustrent l'invention.

### EXEMPLE 1 : Séparation des énantiomères du (3,4-diméthoxy-bicyclo[4.2.0]octa-1,3,5-trién-7-yl)nitrile par chromatographie chirale préparative

Dissoudre 480 mg de composé de formule (I) dans 5 mL de méthanol, injecter sur une colonne Prochrom 50 cm x 50 mm packée sur 25 cm avec 300 g de phase Chiracel OJ à un débit de 80 mL/min et éluer à ce débit dans un mélange heptane/isopropanol (70/30). L'énantiomère de formule (Ia) (configuration (*S*)) est obtenu avec 45,6% de rendement et une pureté énantiomérique de 97,6%.

L'énantiomère de formule (Ib) (configuration (*R*)) est obtenu avec un rendement de 42,2% et une pureté énantiomérique de 99,3%.

### EXEMPLE 2 : [(7S)-3,4-diméthoxybicyclo[4.2.0]octa-1,3,5-trién-7-yl]méthanamine par réduction du (S)-(3,4-diméthoxy-bicyclo[4.2.0]octa-1,3,5-trién-7-yl)nitrile en présence de NaBH₄

Dans un tricol de 125 mL placé sous balayage d'azote et muni d'un réfrigérant, d'un barreau aimanté, d'une garde à CaCl₂ sur l'arrivée d'azote et d'une sonde de température, charger le NaBH₄ (3 éq.) et le THF (10 mL/g). Couler goutte à goutte le TFA (2,97 éq.) à 20-25°C. Additionner la solution de (*S*)-(3,4-diméthoxy-bicyclo[4.2.0]octa-1,3,5-trién-7-yl)nitrile (1éq.) dans le THF (4 mL/g) goutte à goutte. Laisser sous agitation à 20-25 °C pendant la nuit puis couler le milieu réactionnel sur une solution aqueuse d'HCl 0,3 M (0,5 éq.) et laisser agiter à 20-25 °C pendant 1h. Filtrer sur fritté et sous vide, rincer au THF et évaporer le solvant sous pression réduite. Reprendre le brut réactionnel dans du dichlorométhane (20 mL/g), ajouter 10 mL/g d'eau et de la lessive de soude (2 mL/g). Laisser sous agitation 15 minutes puis laisser décanter, laver la phase organique à l'eau, sécher sur MgSO₄ et évaporer le solvant sous pression réduite pour conduire au produit du titre avec 78,8% de rendement et une pureté énantiomérique de 94,4%.

### EXEMPLE 3 : [(7S)-3,4-diméthoxybicyclo[4.2.0]octa-1,3,5-trién-7-yl]méthanamine par réduction du (S)-(3,4-diméthoxy-bicyclo[4.2.0]octa-1,3,5-trién-7-yl)nitrile en présence de palladium sur charbon

Dans un autoclave de 125 mL, charger 1 éq. de (*S*)-(3,4-diméthoxy-bicyclo[4.2.0]octa-1,3,5-trién-7-yl)nitrile, 1 éq. de méthanol HCl 1,12N et 0,1% en masse de palladium sur charbon 5%. Rincer au méthanol (10 mL/g). Purger à l'azote puis à l'hydrogène, agiter à 20°C et hydrogéner sous 30 bars à cette température pendant 5h. Décompresser l'autoclave, filtrer le mélange réactionnel, distiller les solvants sous pression réduite. Reprendre le chlorhydrate obtenu dans du dichlorométhane (20 mL/g), ajouter 10 mL/g d'eau et de la lessive de soude (2 mL/g). Laisser sous agitation 15 minutes puis laisser décanter, laver la phase organique à l'eau, sécher sur MgSO₄ et évaporer le solvant sous pression réduite pour conduire au produit du titre avec 90 % de rendement et une pureté énantiomérique de 95.5 %

### EXEMPLE 4 : (3,4-diméthoxy-bicyclo[4.2.0]octa-1,3,5-trièn-7-yl)nitrile racémique par racémisation du (R)-(3,4-diméthoxy-bicyclo[4.2.0]octa-1,3,5-trién-7-yl)nitrile

Dans un ballon muni d'un réfrigérant et d'une agitation magnétique, charger 100 mg du (*R*)-(3,4-diméthoxy-bicyclo[4.2.0]octa-1,3,5-trién-7-yl)nitrile (0,53 mmol), 5 mL d'isopropanol et 121 mg de DBU (1,5 éq.). Chauffer 2h à 65°C puis laisser revenir à température ambiante. Filtrer pour obtenir le composé du titre.

## Revendications

1. Procédé de résolution optique du (3,4-diméthoxy-bicyclo[4.2.0]octa-1,3,5-trién-7-yl)nitrile de formule (I) dans lequel le mélange racémique ou énantiomériquement enrichi du (3,4-diméthoxy-bicyclo[4.2.0]octa-1,3,5-trién-7-yl)nitrile est séparé en ses deux énantiomères (*S*)-(3,4-diméthoxy-bicyclo[4.2.0]octa-1,3,5-trién-7-yl)nitrile de formule (Ia) et (*R*)-(3,4-diméthoxy-bicyclo[4.2.0]octa-1,3,5-trién-7-yl)nitrile de formule (Ib) par chromatographie chirale.

2. Procédé selon la revendication 1, **caractérisé en ce qu'**il utilise un procédé de séparation multi-colonnes en continu.

3. Procédé selon l'une des revendications 1 ou 2, **caractérisé en ce qu'**il utilise un procédé de chromatographie en lit mobile simulé.

4. Procédé selon l'une des revendications 1 à 3, **caractérisé en ce que** la phase stationnaire utilisée pour la chromatographie chirale comprend un gel de silice imprégné par un polysaccharide fonctionnalisé.

5. Procédé selon l'une des revendications 1 à 4, **caractérisé en ce que** la phase stationnaire utilisée pour la chromatographie chirale comprend un dérivé cellulosique ou amylosique de tris (4-méthylbenzoate) ou de tris (3,5-diméthylphénylcarbamate).

6. Procédé selon l'une des revendications 1 à 5, **caractérisé en ce que** la phase mobile utilisée pour la chromatographie chirale comprend un alcool, un autre solvant organique ou un mélange d'un alcool et d'un autre solvant organique.

7. Procédé selon la revendication 6, **caractérisé en ce que** l'alcool utilisé est l'isopropanol.

8. Procédé selon l'une quelconque des revendications 6 ou 7, **caractérisé en ce que** le solvant organique utilisé est l'heptane ou l'hexane.

9. Procédé selon l'une quelconque des revendications 6 à 8, **caractérisé en ce que** la phase mobile comprend un mélange d'un alcool et d'un autre solvant organique.

10. Procédé selon la revendication 9, **caractérisé en ce que** la phase mobile comprend un mélange d'isopropanol et d'heptane ou un mélange d'isopropanol et d'hexane.

11. Procédé selon la revendication 10, **caractérisé en ce que** la phase mobile comprend un mélange d'isopropanol et d'heptane ou un mélange d'isopropanol et d'hexane dans un rapport variant entre 50:50 et 2:98.

12. Procédé selon l'une des revendications 1 à 11, **caractérisé en ce que** la phase mobile utilisée pour la chromatographie chirale est recyclée.

13. Procédé selon l'une des revendications 1 à 12, **caractérisé en ce que** la chromatographie chirale est réalisée à une température comprise entre 15°C et 40°C.

14. Procédé selon l'une des revendications 1 à 13, **caractérisé en ce que** la résolution optique s'effectue sur le mélange racémique du (3,4-diméthoxy-bicyclo[4.2.0]octa-1,3,5-trién-7-yl)nitrile de formule (I).

15. Procédé selon l'une des revendications 1 à 14 **caractérisé en ce que** l'énantiomère du (3,4-diméthoxy-bicyclo[4.2.0]octa-1,3,5-trién-7-yl)nitrile ciblé est le (*S*)-(3,4-diméthoxybicyclo[4.2.0]octa-1,3,5-trién-7-yl)nitrile de formule (Ia).

16. Procédé selon l'une des revendications 1 à 15, **caractérisé en ce que** l'énantiomère (R) du (3,4-diméthoxy-bicyclo[4.2.0]octa-1,3,5-trién-7-yl)nitrile de formule (Ib) est racémisé et utilisé comme matière première dans le procédé de résolution optique.

17. Composé de formule (Ia) :

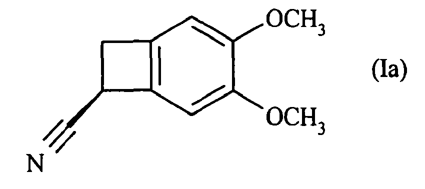

18. Composé de formule (Ib) :

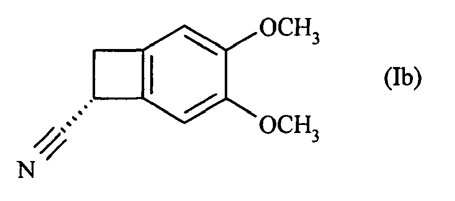

19. Procédé de synthèse du composé de formule (V) : 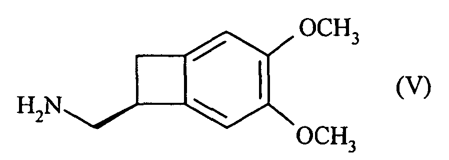 **caractérisé en ce que** le composé de formule (Ia) : 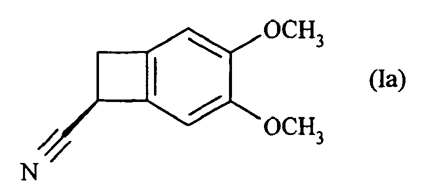 est soumis à une réaction de réduction.

20. Procédé de synthèse selon la revendication 19, **caractérisé en ce que** la réduction du composé de formule (Ia) est effectuée en présence de palladium sur charbon et d'acide chlorhydrique sous atmosphère d'hydrogène ou en présence de tétraborohydrure de sodium et d'acide trifluoroacétique.

21. Procédé de synthèse de l'ivabradine, de ses sels pharmaceutiquement acceptables et de leurs hydrates **caractérisé en ce que** le composé de formule (I) : 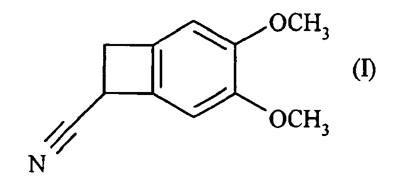 est soumis au procédé de résolution optique de la revendication 1 pour conduire au composé de formule (Ia) : 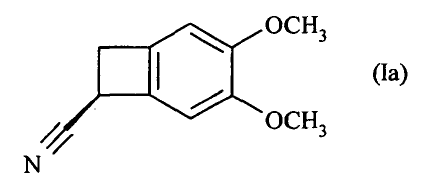 qui est transformé en composé de formule (V) : 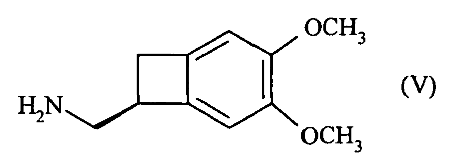 selon le procédé de la revendication 19,
qui est transformé en carbamate de formule (VI) : 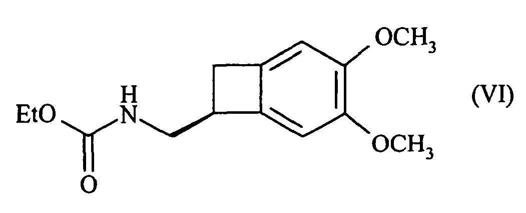 qui est réduit en composé de formule (III) : 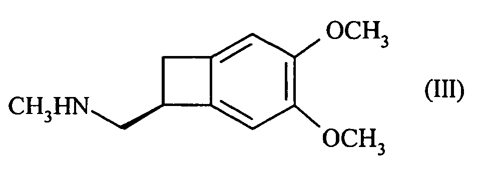 qui est transformé en ivabradine de formule (II) 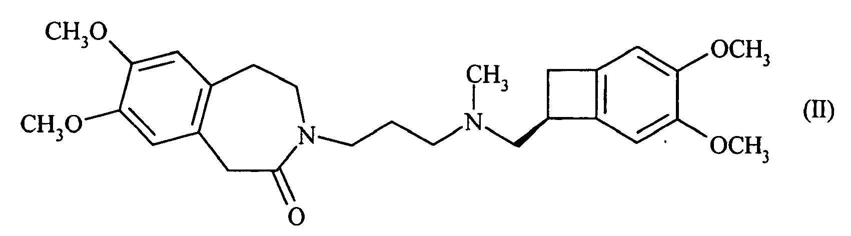 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

## Claims

1. Process for the optical resolution of (3,4-dimethoxy-bicyclo[4.2.0]octa-1,3,5-trien-7-yl)nitrile of formula (I), wherein a racemic or enantiomerically enriched mixture of (3,4-dimethoxy-bicyclo[4.2.0]octa-1,3,5-trien-7-yl)nitrile is separated into its two enantiomers (*S*)-(3,4-dimethoxy-bicyclo[4.2.0]octa-1,3,5-trien-7-yl)nitrile of formula (Ia) and (R)-(3,4-dimethoxy-bicyclo[4.2.0]octa-1,3,5-trien-7-yl)nitrile of formula (Ib) by chiral chromatography.

2. Process according to claim 1, **characterised in that** it uses a continuous multi-column separation process.

3. Process according to one of claims 1 or 2, **characterised in that** it uses a simulated moving bed chromatography process.

4. Process according to one of claims 1 to 3, **characterised in that** the stationary phase used for the chiral chromatography comprises a silica gel impregnated with a functionalised polysaccharide.

5. Process according to one of claims 1 to 4, **characterised in that** the stationary phase used for the chiral chromatography comprises a cellulose or amylose derivative of tris(4-methylbenzoate) or of tris(3,5-dimethylphenylcarbamate).

6. Process according to one of claims 1 to 5, **characterised in that** the mobile phase used for the chiral chromatography comprises an alcohol, another organic solvent or a mixture of an alcohol and another organic solvent.

7. Process according to claim 6, **characterised in that** the alcohol used is isopropanol.

8. Process according to any one of claims 6 or 7, **characterised in that** the organic solvent used is heptane or hexane.

9. Process according to any one of claims 6 to 8, **characterised in that** the mobile phase comprises a mixture of an alcohol and another organic solvent.

10. Process according to claim 9, **characterised in that** the mobile phase comprises a mixture of isopropanol and heptane or a mixture of isopropanol and hexane.

11. Process according to claim 10, **characterised in that** the mobile phase comprises a mixture of isopropanol and heptane or a mixture of isopropanol and hexane in a ratio varying from 50:50 to 2:98.

12. Process according to one of claims 1 to 11, **characterised in that** the mobile phase used for the chiral chromatography is recycled.

13. Process according to one of claims 1 to 12, **characterised in that** the chiral chromatography is carried out at a temperature from 15°C to 40°C.

14. Process according to one of claims 1 to 13, **characterised in that** the optical resolution is carried out on a racemic mixture of (3,4-dimethoxy-bicyclo[4.2.0]octa-1,3,5-trien-7-yl)nitrile of formula (I).

15. Process according to one of claims 1 to 14, **characterised in that** the target enantiomer of (3,4-dimethoxy-bicyclo[4.2.0]octa-1,3,5-trien-7-yl)nitrile is (*S*)-(3,4-dimethoxy-bicyclo-[4.2.0]octa-1,3,5-trien-7-yl)nitrile of formula (Ia).

16. Process according to one of claims 1 to 15, **characterised in that** the (R) enantiomer of (3,4-dimethoxy-bicyclo[4.2.0]octa-1,3,5-trien-7-yl)nitrile of formula (Ib) is racemised and used as starting material in the optical resolution process.

17. Compound of formula (Ia):

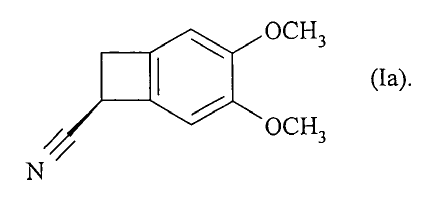

18. Compound of formula (Ib):

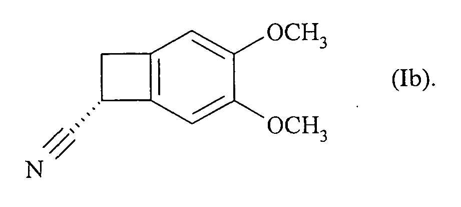

19. Process for the synthesis of the compound of formula (V): 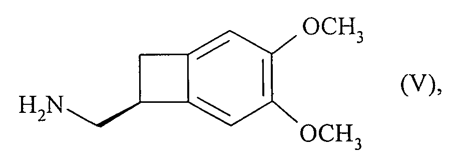 **characterised in that** the compound of formula (Ia): 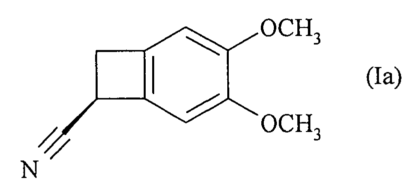 is subjected to a reduction reaction.

20. Synthesis process according to claim 19, **characterised in that** the reduction of the compound of formula (Ia) is carried out in the presence of palladium-on-carbon and HCl under a hydrogen atmosphere or in the presence of sodium tetraborohydride and trifluoroacetic acid.

21. Process for the synthesis of ivabradine, its pharmaceutically acceptable salts, and their hydrates, **characterised in that** the compound of formula (I): 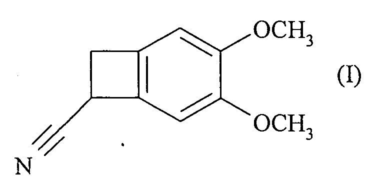 is subjected to the optical resolution process of claim 1 to yield the compound of formula (Ia): 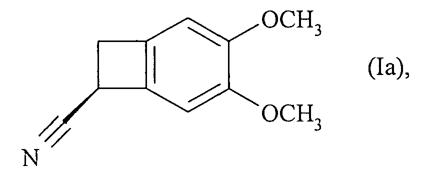 which is converted into the compound of formula (V): 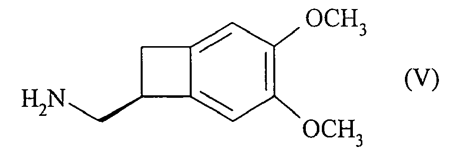 in accordance with the process of claim 19,
which is converted into the carbamate of formula (VI) : 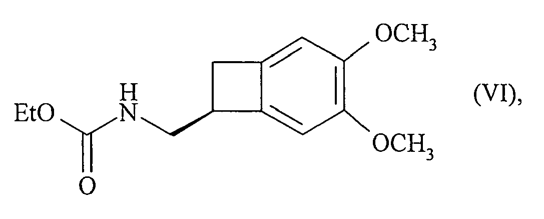 which is reduced to form the compound of formula (III): 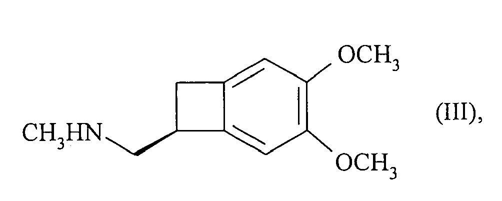 which is converted into ivabradine of formula (II) 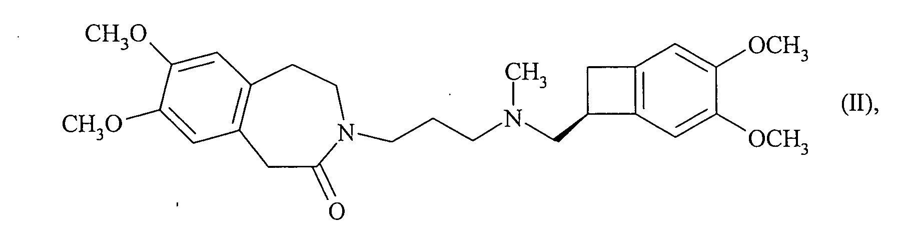 or 3-{3-[{[(7*S*)-3,4-dimethoxybicyclo[4.2.0]octa-1,3,5-trien-7-yl]methyl}(methyl)amino]-propyl}-7,8-dimethoxy-1,3,4,5-tetrahydro-2*H*-3-benzazepin-2-one,
which may optionally be converted into its addition salts with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into their hydrates.

## Patentansprüche

1. Verfahren zur optischen Trennung von (3,4-Dimethoxy-bicyclo[4.2.0]octa-1,3,5-trien-7-yl)-nitril der Formel (I), gemäß dem die racemische oder an dem Enantiomeren von (3,4-Dimethoxy-bieyclo[4.2.0]octa-1,3,5-trien-7-yl)-nitril angereicherte Mischung durch chirale Chromatographie in die beiden Enantiomeren (*S*)-(3,4-Dimethoxybicyclo[4.2.0]octa-1,3,5-trien-7-yl)-nitril der Formel (Ia) und (*R*)-(3,4-Dimethoxybicyclo-[4.2.0]octa-1,3,5-trien-7-yl)-nitril der Formel (Ib) aufgetrennt wird.

2. Verfahren nach Anspruch 1, **dadurch gekennzeichnet, dass** man ein kontinuierliches Multi-Säulen-Trennverfahren anwendet.

3. Verfahren nach einem der Ansprüche 1 oder 2, **dadurch gekennzeichnet, dass** man ein Chromatographieverfahren mit simulierter mobiler Phase anwendet.

4. Verfahren nach einem der Ansprüche 1 bis 3, **dadurch gekennzeichnet, dass** die für die chirale Chromatographie verwendete stationäre Phase ein mit einem funktionalisierten Polysaccharid imprägniertes Silicagel umfasst.

5. Verfahren nach einem der Ansprüche 1 bis 4, **dadurch gekennzeichnet, dass** die für die chirale Chromatographie verwendete stationäre Phase ein Celluose- oder Amylose-Derivat von Tris(4-methylbenzoat) oder Tris(3,5-dimethylphenylcarbamat) umfasst.

6. Verfahren nach einem der Ansprüche 1 bis 5, **dadurch gekennzeichnet, dass** die für die chirale Chromatographie verwendete mobile Phase einen Alkohol, ein anderes organisches Lösungsmittel oder eine Mischung aus einem Alkohol und einem anderen organischen Lösungsmittel umfasst.

7. Verfahren nach Anspruch 6, **dadurch gekennzeichnet, dass** der verwendete Alkohol Isopropanol ist.

8. Verfahren nach einem der Ansprüche 6 oder 7, **dadurch gekennzeichnet, dass** das verwendete organische Lösungsmittel Heptan oder Hexan ist.

9. Verfahren nach einem der Ansprüche 6 bis 8, **dadurch gekennzeichnet, dass** die mobile Phase eine Mischung aus einem Alkohol und einem anderen organischen Lösungsmittel umfasst.

10. Verfahren nach Anspruch 9, **dadurch gekennzeichnet, dass** die mobile Phase eine Mischung aus Isopropanol und Heptan oder eine Mischung aus Isopropanol und Hexan umfasst.

11. Verfahren nach Anspruch 10, **dadurch gekennzeichnet, dass** die mobile Phase eine Mischung aus Isopropanol und Heptan oder eine Mischung aus Isopropanol und Hexan in einem Verhältnis von zwischen 50 : 50 und 2 : 98 umfasst.

12. Verfahren nach einem der Ansprüche 1 bis 11, **dadurch gekennzeichnet, dass** die für die chirale Chromatographie verwendete mobile Phase recyclisiert wird.

13. Verfahren nach einem der Ansprüche 1 bis 12, **dadurch gekennzeichnet, dass** die chirale Chromatographie bei einer Temperatur zwischen 15 °C und 40 °C durchgeführt wird.

14. Verfahren nach einem der Ansprüche 1 bis 13, **dadurch gekennzeichnet, dass** die optische Trennung der racemischen Mischung von (3,4-Dimethoxy-bicyclo[4.2.0]octa-1,3,5-trien-7-yl)-nitril der Formel (I) durchgeführt wird.

15. Verfahren nach einem der Ansprüche 1 bis 14, **dadurch gekennzeichnet, dass** das angestrebte Enantiomere des (3,4-Dimethoxy-bicyclo[4.2.0]octa-1,3,5-trien-7-yl)-nitrils (*S*)-(3,4-Dimethoxy-bicyclo[4.2.0]octa-1,3,5-trien-7-yl)-nitril der Formel (Ia) ist.

16. Verfahren nach einem der Ansprüche 1 bis 15, **dadurch gekennzeichnet, dass** das (*R*)-Enantiomere des (3,4-Dimethoxy-bicyclo[4.2.0]octa-1,3,5-trien-7-yl)-nitrils der Formel (Ib) racemisiert und als Ausgangsmaterial bei dem Verfahren zur optischen Trennung eingesetzt wird.

17. Verbindung der Formel (Ia):

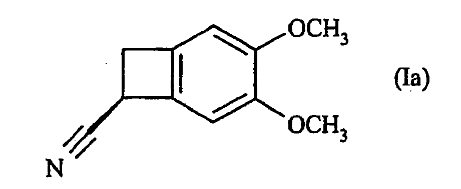

18. Verbindung der Formel (Ib):

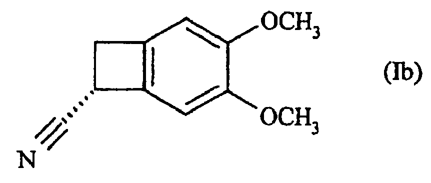

19. Verfahren zur Synthese der Verbindung der Formel (V): 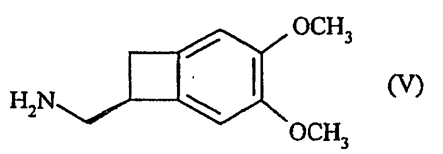 **dadurch gekennzeichnet, dass** man die Verbindung der Formel (Ia): 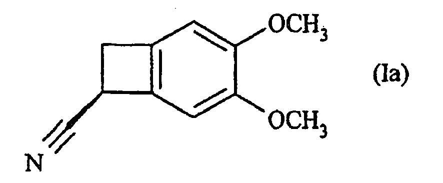 einer Reduktionsreaktion unterwirft.

20. Syntheseverfahren nach Anspruch 19, **dadurch gekennzeichnet, dass** die Reduktion der Verbindung der Formel (Ia) in Gegenwart von Palladium-auf-Kohlenstoff und Chlorwasserstoffsäure in einer Wasserstoffatmosphäre oder in Gegenwart von Natriumtetraborhydrid und Trifluoressigsäure durchgeführt wird.

21. Verfahren zur Synthese von Ivabradin, seinen pharmazeutisch annehmbaren Salzen und ihren Hydraten, **dadurch gekennzeichnet, dass** die Verbindung der Formel (I): 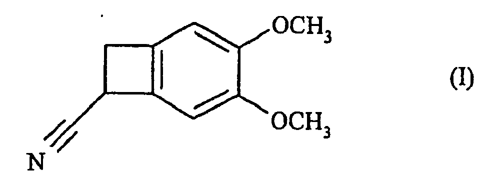 dem optischen Trennungsverfahren gemäß Anspruch 1 unterworfen wird zur Bildung der Verbindung der Formel (Ia): 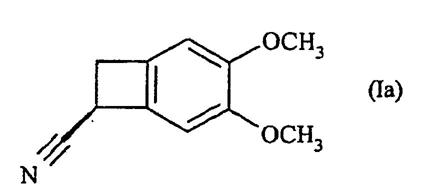 die nach dem Verfahren von Anspruch 19 in die Verbindung der Formel (V) umgewandelt wird: 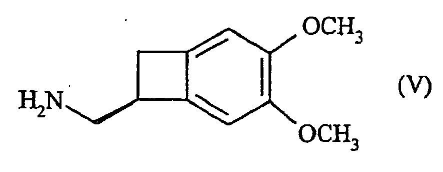 welche in das Carbamat der Formel (VI) überführt wird: 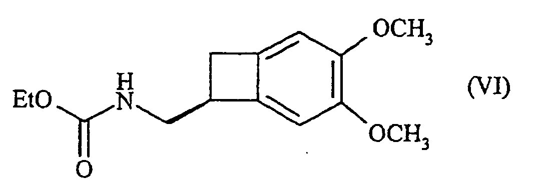 welches zu der Verbindung der Formel (III) reduziert wird: 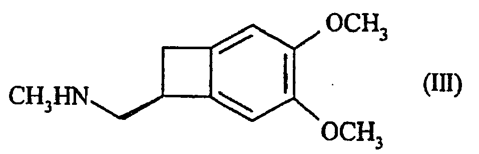 welche in Ivabradin der Formel (II): 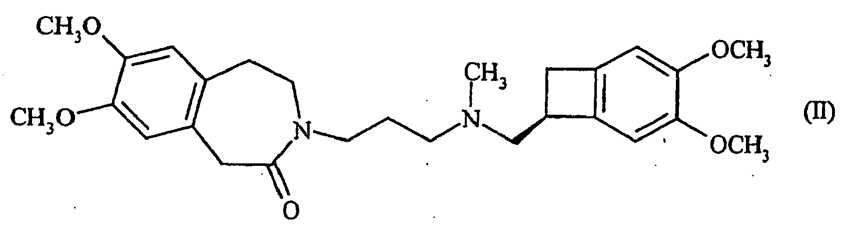 oder 3-{3-[{[(7*S*)-3,4-Dimethoxybicyclo[4.2.0]octa-1,3,5-trien-7-yl]-methyl](methyl)-amino]-propyl}-7,8-dimethoxy-1,3,4,5-tetrahydro-2*H*-3-benzazepin-2-on umgewandelt wird,
welches gegebenenfalls in seine Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und deren Hydrate umgewandelt werden kann.
